# EUROPEAN PATENT SPECIFICATION

(11) **EP 3 085 333 B1**
(45) Date of publication and mention of the grant of the patent: **05.06.2019**
(21) Application number: 15382203.6
(22) Date of filing: 23.04.2015
(51) Int. Cl.: A61F 2/00

(54) **REMOTE-CONTROLLED EXTRA-URETRAL OCCLUSIVE VALVE**
FERNGESTEUERTES EXTRAURETHRALES OKKLUSIONSVENTIL
VALVE D'OCCLUSION EXTRA-URÉTRALE TÉLÉCOMMANDE

(43) Date of publication of application: 26.10.2016
(73) Proprietor: Arquimea Ingenieria, S.L.U., 28919 Leganes (Madrid) (ES)
(72) Inventor: RIVERA SORIA, Cayetano, 28919 Leganés (Madrid) (ES); CABÁS ORMAECHEA, Ramiro, 28919 Leganés (Madrid) (ES); TELLEZ MARTINEZ-FORNÉS, Miguel, 28919 Leganés (Madrid) (ES); MORENO LORENTE, Luis Enrique, 28919 Leganés (Madrid) (ES)
(74) Representative: Carpintero Lopez, Francisco

(56) References cited:
- CN-A- 102 166 139
- US-A- 3 926 175
- US-A1- 2004 242 956
- US-A1- 2006 047 180

## Description

### Technical Field of the Invention

The object of the present invention is a remote-controlled extra-urethral occlusive valve to prevent losses of urine in patients with urinary incontinence.

More specifically, the object of the occlusive valve of the invention is to occlude the urethra by means of a shape memory alloy or SMA element which allows operating on said valve in a simple manner and through a mechanism that is safe, with a reduced number of elements to prevent failures, and efficient from the viewpoint of achieving correct occlusion of the urethra with minimum power consumption.

### Background of the Invention

As known, there are several types of urinary incontinence, such as urge incontinence, overflow incontinence and stress incontinence.

This latter type of incontinence is treated in many different ways according to the case, for example by means of containment mechanisms such as diapers and the like, pharmacological treatment, pelvic floor or Kegel exercises, vaginal cones, electrical stimulation and urethral occlusive devices.

These urethral occlusive devices are divided into intra-urethral or extra-urethral devices depending on whether they close the urethra by pressing from the outside or through a valve mechanism acting from the inside, respectively, and in the latter case, these devices furthermore involve surgical treatment.

As known, one of the main drawbacks of existing intra-urethral occlusive devices is that they cannot be applied to treat incontinence in men due to the characteristics of man urethra. The few studies in this regard indicated that, although 80-85% of patients showed significant improvement in terms of leakages, there was a high percentage of patients who suffered placement discomforts, difficulty in securing the device and side effects, which resulted in complications such as asymptomatic bacteriuria, hematuria, repetitive urinary tract infections and mucosal irritation by the device. Furthermore, the cost of this treatment is relatively high as it requires replacing the device several times a day.

As regards artificial extra-urethral occlusive devices, e.g. US 2006/0047180, the device most widely used today is (AMS800) developed by the company AMS based on a periurethral cuff similar to cuffs for taking blood pressure which is kept inflated at rest and is deflated when the patient need to urinate. Nevertheless, this device also has problems such as in virtually all series with follow-up close to 5 years initial results of continence less than 80% (about 70%) and, furthermore, worsening of the results of continence over time, in addition to a 30% of patients requiring at least one re-intervention in the first 5 years due to mechanical failures or nonmechanical complications (erosion or infection), can be seen. On the other hand, it has been observed that all patients have to a greater or lesser extent urethral atrophy problems due to pressure, the system loses pressure and therefore efficacy after 4 months, requires certain skill for use thereof, has a high cost and, finally, poses considerable risk of infection and rejection.

To solve some of these problems the apparatus described in patent document EP1253880 is disclosed comprising an electric motor to more or less compress the urethra, controllable by means of an operating device for changing compression in the urethra, as well as a control device for controlling a source of energy, which may or may not be implanted in the body, from outside the patient's body. However, this device has drawbacks inherent to such devices, such as for example, its size and complexity, large number of components, cost, etc.

There are in the art other systems such as those shown in WO2011107638 or US2004/0242956 providing alternative to this document.

More specifically, the first document WO2011107638, belonging to the same owners, discloses a urethral occlusive device controlled by pressure external to the urethra but internal to the patient's body that is based on a shape memory alloy or SMA material, an electronic control system allowing the patient to handle the device easily and remotely. This urethral occluder comprises a casing containing a fluid, all the walls thereof are rigid except for one that is closed by a flexible membrane arranged for coming into contact with the urethra and applying a suitable pressure thereon preventing the passage of urine at the moment of retention, a rigid casing for focusing all the force the system attains towards the membrane, an element which will change its shape upon activating the SMA material from the outside, a recovery mechanism so that the device applies a suitable pressure when the SMA material is deactivated, which SMA material has a suitable shape for compressing and expanding, varying the inner volume of the occluder such that the fluid transmits the pressure on the flexible membrane depending on the movement of said shape memory alloy part.

The other patent document US2004/0242956 shows an external mechanism acting as an artificial sphincter by means of using several metal plates attached to other ceramic plates like a sandwich and, in turn, the entire assembly at the ends thereof to an element encircling the duct to be occluded. Therefore, the assembly bends when applying electricity pushing an element which acts on said duct to be occluded closing it. When the electric current ceases, the plates recover their position releasing the duct again and allowing passage of fluid therethrough.

The first patent document, however, has some drawbacks derived from its particular structure. Specifically, the two fundamental drawbacks are the following:
a) The fact that in said patent document the net force and movement generated by the change in the SMA material is transmitted to a bag of water which regulates and distributes pressure means that most of the work performed by the device is lost in deforming and adapting that bag without really having any actual effect on the urethra;
b) On the other hand, in the use of SMA materials which must produce work, such as the case of closing or opening a duct, in this case the urethra, the shape or morphology of the element made of an SMA material is essential to achieve an optimal balance between performance and volume. In this case, however, an element made of an SMA material with a spring shape is used, which even if it has a good efficiency in terms of work performed, on the other hand, it occupies a considerable space in the direction of said work, which involves a drawback in that type of applications where the size of the device to be implanted in the patient's body is critical.

The system described in the other patent document US2004/0242956 does not have the drawbacks of WO2011107638 described above since it eliminates, on one hand, the drawbacks relating to efficiency by not having an intermediate element such as a fluid and, on the other hand, the drawbacks relating to the volume occupied by not using a spring-shaped SMA element.

Specifically, the device shown in US2004/0242956 uses as an element for performing work a bimorph device formed by a flexible metal foil sandwiched between two piezoelectric elements, where said bimorph device is working under bending, with two supports at the ends thereof and a central point for applying force in a position perpendicular to the movement to be achieved.

Although this device successfully reduces the volume by not using a spring such as in WO2011107638, it has, however, a significant drawback described below.

Specifically, said patent document shows that the actuator element is formed by foils or bars that bend when an electric current acts on them, but they are either fitted at one end (so they have 3 restricted degrees of freedom), which allows extensive travel but very little strength, or are fitted at both ends (with 6 restricted degrees of freedom) whereby there is great strength which is unnecessary in this application and a travel that is insufficient for there to be enough difference between the open and closed position, i.e., to achieve perfect closing of the urethra of the patient.

Therefore, there is still a need in the art of a low-cost artificial sphincter with results exceeding those obtained by those used today, reducing the complication rate and the high re-intervention rate.

### Description of the Invention

The present invention solves the aforementioned drawbacks of the state of the art since it uses a shape memory alloy or SMA actuator element allowing a performance similar to the spring configuration and an optimum efficacy while occupying a minimum volume, furthermore simplifying the design as it does not need complicated mechanisms or intermediate mechanisms while at the same time achieving perfect occlusion of the urethra, all this while occupying a smaller space resulting in a device having small dimensions that can be perfectly implanted in the patient.

Therefore, the present invention relates to an extra-urethral occlusive valve, i.e., occluding or closing the urethra from outside same, embracing it, so it is implanted inside the patient's body and being remotely controllable and operable by him.

Basically, the valve of the invention is based on a shape memory alloy element that it is controlled by an electronic control system and has a battery or other equivalent system for powering said control system and heating the SMA material during activation thereof by means of electric current.

Specifically, the shape memory alloy element is made in the present invention in a foil- or plate-type configuration. As a result of this configuration, very large movements are achieved compared to the dimension occupied by the element in the direction in which the movement occurs. By way of example, if in a spring configuration said spring must occupy 10 mm to achieve 5 mm of movement, to achieve that same movement the foils occupy less than 1 mm, furthermore without compromising the force that they are capable of applying, which is similar to that provided by the spring. In other words, by means of using plates or foils efficiency results (force applied) virtually like those in a spring configuration are obtained, but by using a significantly lower volume.

Furthermore, given that it is implanted internally, it has an external activation system sending instructions to the control system wirelessly, which allows the patient to remotely handle same in an easy, comfortable and simple manner.

More specifically, the extra-urethral valve of the invention comprises a casing which is placed embracing the urethra of the user such that it is facing a hole said casing has on its surface, which hole allows passage of a projection which, as a result of the actuation mechanism, enters and comes out of said casing constricting or releasing the urethra and where said projection forms part of or is integrally attached to a moving part housed inside the casing which can slide longitudinally in a guided manner and operated by means of the shape memory alloy or SMA element.

Therefore, in the resting state, said projection is outside the casing pressing on the urethra and occluding it, whereas, when the valve is actuated by the user, said projection is retracted being introduced in the casing and therefore releasing the urethra.

This simple actuation of the valve of the invention is achieved by means of two elements located inside said casing and acting on the moving part, specifically:
- An elastic body responsible for pressing on said moving part so that the projection protrudes from the casing pressing on the urethra in the resting position of the valve; and
- A shape memory alloy element such that, when being activated by the user, it applies force to retract the moving part and thereby the projection into the casing.

Therefore, in the resting state, the shape memory alloy element has a low rigidity and is readily deformable, so the force of the elastic body acting on the moving part predominates to draw the projection closing the urethra out of the casing, sealing it and preventing the urine flow.

When the user activates the valve, the temperature of the SMA element and therefore its rigidity increases, calculated to overcome the force of the elastic body and drive the moving part such that the projection it has is introduced inside the casing, which entails releasing the urethra and allowing the urine flow.

As heat gradually dissipates from the shape memory alloy element, the latter loses rigidity and therefore the force of the elastic body prevails again, causing the projection associated with the moving part to protrude progressively to the initial resting position, again pressing on the urethra and closing the urine flow.

Finally and essentially, the valve of the invention comprises a shape memory alloy element such that it works under bending, i.e., said shape memory alloy element has an elongated shape, such as for example, a foil or a bar which, being supported at the ends thereof, bends such that the point located at the center thereof transmits the force to the element on which they act, in this case the moving part.

However, unlike the examples shown in the state of the art, in the valve of the invention the ends of the shape memory alloy element are free, only supported but not secured or fitted, i.e., so the movement thereof is only restricted in two degrees of freedom, allowing both movement and rotation at said end support points.

More specifically, by allowing these movements and rotations all the deformation of the material of the shape memory alloy element always occur by bending, without thrust or compression components, allowing being able to take advantage of the change in geometry in a more efficient manner without exceeding the critical percentages that can damage the material.

Balance is thereby achieved where the force is high and a very large movement of the shape memory alloy element subject to bending is achieved at the same time as it is not restricted by fittings. This configuration is particularly effective when work is performed around the area of maximum efficiency which happens when the central point of the SMA element is moved a distance equal to half the distance between shafts, central point which, as mentioned, transmits the force to the moving part.

A more efficient way of working is thereby achieved by attaining better ratios between the space occupied by said element and the movement and applied force necessary for occluding the urethra, all this furthermore taking into account that, as mentioned, working in critical situations which may damage the shape memory alloy element is prevented.

### Description of the Drawings

To complement the description that is being made and for the purpose of aiding to better understand the features of the invention according to a preferred practical embodiment thereof, a set of drawings is attached as an integral part of said description in which the following has been depicted with an illustrative and non-limiting character:

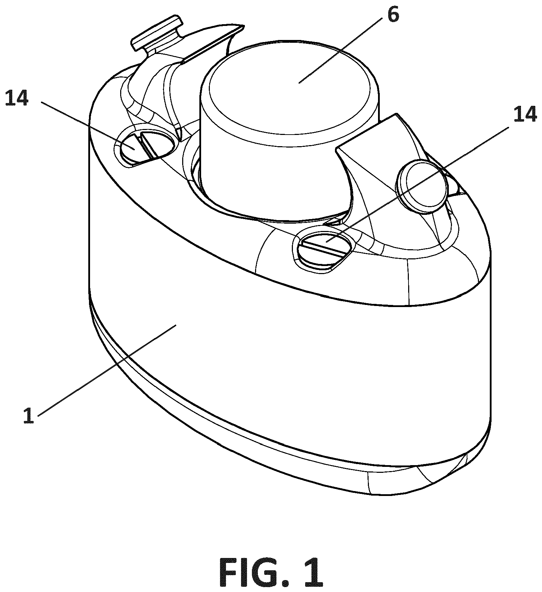

Figure 1 shows a perspective view of the valve of the invention in resting position, with the projection intended for pressing on the urethra protruding outwards, although said urethra has not been shown.
Figure 2 shows an elevational sectioned view of the valve of the invention in the resting position, i.e., while the urethra is being pressed on.
Figure 3 shows an elevational sectioned view of the valve of the invention in an operative position, i.e., releasing the urethra due to the action of the shape memory alloy element operated by the user bearing the implanted element.

### Preferred Embodiment of the Invention

In view of the described drawings, an embodiment of the remote-controlled extra-urethral valve of the invention can be seen, comprising the following:
- A casing (1) which is placed embracing the urethra (2) with the help of a belt (3) or the like made of a biocompatible material.

Said casing (1) can be formed by one or more parts, in which case said parts are attached by means of screws (14) or any other conventional means while at the same time having means such as an O-ring (4), for example, for assuring leak-tightness which is essential as it is implanted inside the body,

According to the embodiment, said casing (1) in turn internally comprises:
- A moving part (5) which can slide longitudinally in a guided manner;
- A projection (6) which forms part of or is integrally attached to the moving part;
- A hole through which said projection (6) can extend outwards pressing on the urethra (2);
- An elastic body (7), such as a spring, for example, responsible for pressing on said moving part (5) so that the projection (6) protrudes from the casing (1) pressing on the urethra (2) in the resting position of the valve; and
- A shape memory alloy element (8) which, when being activated by the user, applies force such that it overcomes the resistance of the elastic body (7) and retracts the moving part into the casing. In contrast, when said shape memory alloy element (8) is no longer activated, the latter loses its rigidity, the force of the elastic body (7) prevailing and causing the projection (6) pressing on the urethra (2) to protrude through the hole of the casing (1).

According to the embodiment of the invention, the shape of the shape memory alloy element (8) is such that it works under bending, for example a plate, a foil or a bar such that, when activated, it bends with its ends (9) being supported, transmitting force to the moving part (5).

Furthermore, according to said embodiment, said ends (9) are freely supported, without being secured or fitted on respective free rotating shafts (10).

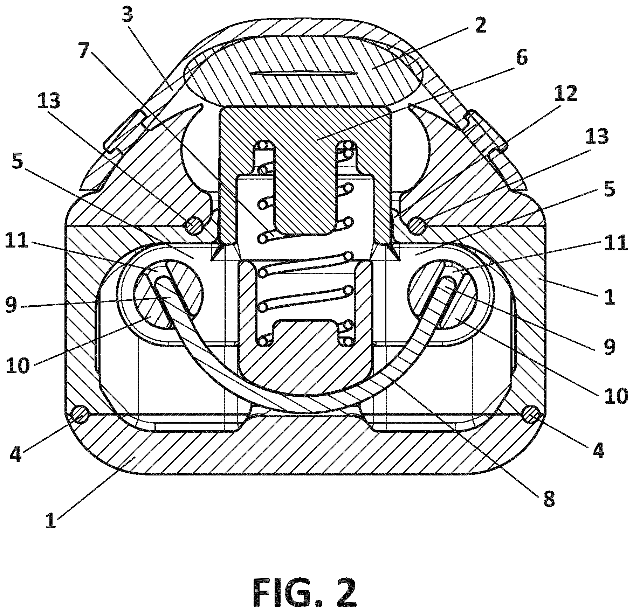

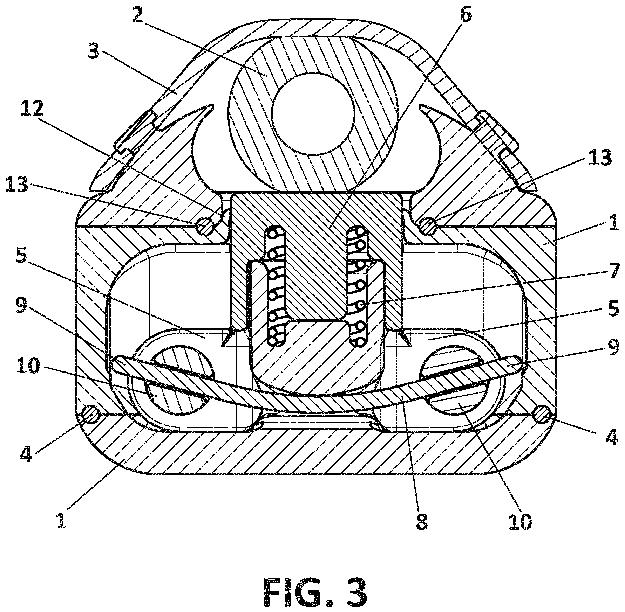

More specifically, each of said shafts (10) comprises a groove (11) serving as a housing for the mentioned ends (9) of the shape memory alloy element (8), housing which allows relative movement of said ends therethrough during bending of the shape memory alloy element (8), as can be seen in Figures 2 and 3, showing the two positions of the valve, at rest and in an operative position, respectively, and showing how the position of said grooves (11) has changed to allow re-orientating the support they provide to said shape memory alloy element (8) holding it at all times.

Therefore, by means of said grooves movement of the ends (9) of the shape memory alloy element (8) is allowed and, as a result of the fact that the shafts (10) comprising said grooves can rotate, deformation of the shape memory alloy element (8) always occur by bending, without thrust or compression components, as explained above.

On the other hand, instead of the arrangement described and shown in Figures 2 and 3 in which the shafts (10) are fixed to the moving part (5) and the shape memory alloy element (8) has at least one attachment point to the casing (1), another possible alternative embodiment, which is not depicted, would be that in which said shafts (10) were fixed to the casing (1) but the shape memory alloy element (8) was fixed to the moving part (5).

Nevertheless, in both embodiments the mentioned shafts (10) have freedom of rotation to allow movement of the ends (9) of the shape memory alloy element (8) as it bends upon activation thereof.

Said activation can furthermore be done either by heating the shape memory alloy element (8) by circulating a current therethrough, or indirectly using a heating element which in turn transfers said heat thereto.

Finally, as can be seen in the drawings, there is placed surrounding the hole through which the projection (6) extends outwards an elastic membrane (12) which is secured in position with the help of a second O-ring (13), the entire assembly then being fixed by means of screws (14).

## Claims

1. Remote-controlled extra-urethral occlusive valve, comprising:
- a casing (1) embracing the urethra (2),
- a moving part (5) which can slide longitudinally in a guided manner inside the casing (1) and which has a projection (6) for pressing on the urethra (2) through a hole of the casing (1),
- an elastic body (7) which presses on the moving part (5) in the resting position so that the projection (6) in turn presses on the urethra (2),
- a shape memory alloy element (8) which, when activated by heating, bends and overcomes the resistance of the elastic body (7) retracting the moving part into the casing and releasing the urethra (2), and
- two rotating shafts (10);
**characterized in that**
- the shape memory alloy element (8) comprises two ends (9), and
- the rotating shafts (10) comprise a groove (11) serving as a housing for the ends (9) of the shape memory alloy element (8);
where the two ends (9) are supported, without being secured or fitted, on respective the free rotating shafts (10), such that during bending of the shape memory alloy element (8) movement of said ends (9) on the shafts (10) is allowed.

2. Remote-controlled extra-urethral occlusive valve according to claim 1, **characterized in that** the shafts (10) are fixed to the moving part (5) with freedom of rotation and the shape memory alloy element (8) has at least one attachment point to the casing (1).

3. Remote-controlled extra-urethral occlusive valve according to claim 1, **characterized in that** the shafts (10) are fixed to the casing (1) with freedom of rotation and the shape memory alloy element (8) is fixed to the moving part (5).

4. Remote-controlled extra-urethral occlusive valve according to any of the preceding claims, **characterized in that** it comprises a belt (3) to help the casing (1) embrace the urethra (2).

5. Remote-controlled extra-urethral occlusive valve according to any of the preceding claims, **characterized in that** the casing (1) is formed by several parts attached to one another and comprises means for assuring leak-tightness.

6. Remote-controlled extra-urethral occlusive valve according to claim 5, **characterized in that** the means for assuring leak-tightness are formed by an O-ring (4).

7. Remote-controlled extra-urethral occlusive valve according to any of the preceding claims, **characterized in that** it comprises an elastic membrane (12) surrounding the hole through which the projection (6) extends utwards.

8. Remote-controlled extra-urethral occlusive valve according to claim 8, **characterized in that** the elastic membrane (12) is secured with the help of a second O-ring (13).

9. Remote-controlled extra-urethral occlusive valve according to any of the preceding claims, **characterized in that** the shape memory alloy element (8) is a plate, a foil or a bar.

## Patentansprüche

1. Ferngesteuertes extraurethrales Okklusionsventil mit:
- einem die Urethra (2) umschließenden Gehäuse (1),
- einem Bewegungsteil (5), das geführt in Längsrichtung in dem Gehäuse (1) gleiten kann, und das einen Vorsprung (6) zum Drücken gegen die Urethra (2) durch ein Loch in dem Gehäuse (1) aufweist,
- einem elastischen Körper (7), der in der Ruheposition gegen das Bewegungsteil (5) drückt, so dass der Vorsprung (6) seinerseits gegen die Urethra (2) drückt,
- einem Formgedächtnislegierungselement (8), das sich, wenn es durch Erwärmen aktiviert wird, biegt und den Widerstand des elastischen Körpers (7) überwindet, welcher das Bewegungsteil in das Gehäuse zurückzieht und die Urethra (2) freigibt, und
- zwei Drehwellen (10),
**dadurch gekennzeichnet, dass**
- das Formgedächtnislegierungselement (8) zwei Enden (9) aufweist, und
- die Drehwellen (10) eine Nut (11) aufweisen, welche als ein Gehäuse für die Enden (9) des Formgedächtnislegierungselements (8) dient,
wobei die beiden Enden (9) ohne befestigt oder eingesetzt zu sein jeweils an den freien Drehwellen (10) derart gelagert sind, dass während des Biegens des Formgedächtnislegierungselements (8) eine Bewegung der Enden (9) an den Wellen (10) möglich ist.

2. Ferngesteuertes extraurethrales Okklusionsventil nach Anspruch 1, **dadurch gekennzeichnet, dass** die Wellen (10) an dem Bewegungsteil (5) mit Drehfreiheit befestigt sind und das Formgedächtnislegierungselement (8) mindestens einen Punkt zur Befestigung an dem Gehäuse (1) aufweist.

3. Ferngesteuertes extraurethrales Okklusionsventil nach Anspruch 1, **dadurch gekennzeichnet, dass** die Wellen (10) an dem Gehäuse (1) mit Drehfreiheit befestigt sind und das Formgedächtnislegierungselement (8) an dem Bewegungsteil (5) befestigt ist.

4. Ferngesteuertes extraurethrales Okklusionsventil nach einem der vorhergehenden Ansprüche, **dadurch gekennzeichnet, dass** es einen Gurt (3) aufweist, um das Gehäuse (1) beim Umschließen der Urethra (2) zu unterstützen.

5. Ferngesteuertes extraurethrales Okklusionsventil nach einem der vorhergehenden Ansprüche, **dadurch gekennzeichnet, dass** das Gehäuse (1) durch mehrere aneinander befestigte Teile gebildet ist und Einrichtungen aufweist, die Dichtigkeit gewährleisten.

6. Ferngesteuertes extraurethrales Okklusionsventil nach Anspruch 5, **dadurch gekennzeichnet, dass** die Einrichtungen zur Gewährung der Dichtigkeit durch einen Dichtungsring (4) gebildet sind.

7. Ferngesteuertes extraurethrales Okklusionsventil nach einem der vorhergehenden Ansprüche, **dadurch gekennzeichnet, dass** es eine elastische Membran (12) aufweist, welche das Loch umgibt, durch welches sich der Vorsprung (6) nach außen erstreckt.

8. Ferngesteuertes extraurethrales Okklusionsventil nach Anspruch 8, **dadurch gekennzeichnet, dass** die elastische Membran (12) mittels eines zweiten Dichtungsrings (13) befestigt ist.

9. Ferngesteuertes extraurethrales Okklusionsventil nach einem der vorhergehenden Ansprüche, **dadurch gekennzeichnet, dass** das Formgedächtnislegierungselement (8) eine Platte, eine Folie oder eine Stange ist.

## Revendications

1. Valve d'occlusion extra-urétrale télécommandée comprenant :
- un boîtier (1) enveloppant l'urètre (2),
- une pièce mobile (5) qui peut coulisser longitudinalement d'une manière guidée à l'intérieur du boîtier (1) et qui a une saillie (6) pour appuyer sur l'urètre (2) à travers un trou du boîtier (1),
- un corps élastique (7) qui appuie sur la pièce mobile (5) dans la position de repos de façon que la saillie (6) à son tour appuie sur l'urètre (2),
- un élément en alliage à mémoire de forme (8) qui, lorsqu'il est activé par chauffage, fléchit et surmonte la résistance du corps élastique (7) rétractant la pièce mobile dans le boîtier et libérant l'urètre (2), et
- deux arbres rotatifs (10) ;
**caractérisée en ce que**
- l'élément en alliage à mémoire de forme (8) comprend deux extrémités (9), et
- les arbres rotatifs (10) comprennent une rainure (11) servant de boîtier pour les extrémités (9) de l'élément en alliage à mémoire de forme (8) ;
où les deux extrémités (9) sont supportées, sans être fixées ou ajustées, sur les arbres rotatifs respectifs (10), de façon que, durant le fléchissement de l'élément en alliage à mémoire de forme (8), un mouvement desdites extrémités (9) sur les arbres (10) soit permis.

2. Valve d'occlusion extra-urétrale télécommandée selon la revendication 1, **caractérisée en ce que** les arbres (10) sont fixés à la pièce mobile (5) avec une liberté de rotation et l'élément en alliage à mémoire de forme (8) a au moins un point d'attachement au boîtier (1).

3. Valve d'occlusion extra-urétrale télécommandée selon la revendication 1, **caractérisée en ce que** les arbres (10) sont fixés au boîtier (1) avec une liberté de rotation et l'élément en alliage à mémoire de forme (8) est fixé à la pièce mobile (5).

4. Valve d'occlusion extra-urétrale télécommandée selon l'une quelconque des revendications précédentes, **caractérisée en ce qu'**elle comprend une courroie (3) pour aider le boîtier (1) à envelopper l'urètre (2).

5. Valve d'occlusion extra-urétrale télécommandée selon l'une quelconque des revendications précédentes, **caractérisée en ce que** le boîtier (1) est formé de plusieurs parties attachées les unes aux autres et comprend des moyens pour assurer une étanchéité vis-à-vis des fuites.

6. Valve d'occlusion extra-urétrale télécommandée selon la revendication 5, **caractérisée en ce que** les moyens pour assurer une étanchéité vis-à-vis des fuites sont formés d'un joint torique (4).

7. Valve d'occlusion extra-urétrale télécommandée selon l'une quelconque des revendications précédentes, **caractérisée en ce qu'**elle comprend une membrane élastique (12) entourant le trou à travers lequel la saillie (6) s'étend vers le haut.

8. Valve d'occlusion extra-urétrale télécommandée selon la revendication 8, **caractérisée en ce que** la membrane élastique (12) est fixée à l'aide d'un deuxième joint torique (13).

9. Valve d'occlusion extra-urétrale télécommandée selon l'une quelconque des revendications précédentes, **caractérisée en ce que** l'élément en alliage à mémoire de forme (8) est une plaque, une feuille ou une barre.
